# FASCICULE DE BREVET EUROPEEN

(11) **EP 3 021 774 B1**
(45) Date de publication et mention de la délivrance du brevet: **30.08.2017**
(21) Numéro de dépôt: 14747074.4
(22) Date de dépôt: 02.07.2014
(51) Int. Cl.: A61B 17/84, A61B 17/86

(54) **VIS D'OSTÉOSYNTHÈSE**
OSTEOSYNTHESESCHRAUBE
OSTEOSYNTHESIS SCREW

(30) Priorité: 18.07.2013 FR 1357084
(43) Date de publication de la demande: 25.05.2016
(73) Titulaire: Fumex, Laurent, Madison, CT 06443 (US); Masseglia, Thierry, 83130 La Garde (FR)
(72) Inventeur: Fumex, Laurent, Madison, CT 06443 (US); Masseglia, Thierry, 83130 La Garde (FR)
(74) Mandataire: L'Helgoualch, Jean
(86) Numéro de dépôt international: PCT/FR2014/051694
(87) Numéro de publication internationale: WO 2015/007975

(56) Documents cités:
- WO-A1-91/09572
- DE-U1-202012 005 594
- US-A- 4 640 271
- US-A- 5 743 912

## Description

La présente invention concerne un dispositif d'ostéosynthèse destiné à maintenir en distraction ou compression des fragments d'un os fracturé.

On connaît de nombreux dispositifs de type vis d'ostéosynthèse, broche filetée ou non, agrafe, vis ou broche se bloquant dans une plaque, permettant de réaliser une ostéosynthèse d'un os fracturé.

Le brevet US7037309 décrit une vis auto-taraudante canulée à deux filetages à pas différents qui permet la compression d'un os fracturé. Le principe de cette vis est que la différence entre le pas du filetage distale et le pas du filetage proximal permet une compression de l'os lors du vissage de la vis. Cependant, la valeur de compression n'est pas maîtrisable car elle dépend de la qualité de réduction du foyer de fracture ainsi que de la qualité de l'os. De plus, une importante gamme de vis est nécessaire pour répondre aux différentes longueurs des os fracturés.

Le brevet US20110218580 décrit une vis à deux filetages à pas différents dont la tête filetée se bloque dans une plaque. Ce dispositif permet le maintien d'une fracture mais il ne peut être enfoui dans l'os.

Le brevet US7641677 décrit une broche filetée comportant une bague qui se visse sur le filetage de la broche. Ce dispositif permet le maintien et la compression du fragment d'os fracturé mais ne peut être enfoui.

Le brevet WO2009/103886 décrit une broche filetée qui traverse une douille qui, tout en se bloquant dans une plaque, bloque la broche en translation. Ce dispositif permet le maintien d'une fracture mais il ne peut être enfoui dans l'os. Le modèle d'utilité DE 20 2012 005 594 U1 décrit un dispositif d'ostéosynthèse selon le préambule de la revendication 1. Ces dispositifs permettent effectivement le maintien et/ou la compression des fragments d'un os fracturé. Toutefois, ils ne permettent pas au praticien de choisir, en fonction du type de fracture, d'écarter, de comprimer ou maintenir un fragment osseux.

La présente invention a pour objet de fournir un dispositif permettant au praticien le réglage manuel de la distraction ou de la compression, le maintien d'un fragment osseux, d'être enfoui dans l'os, de réduire significativement la gamme d'implants et une mise en place au moyen d'outils usuels.

Le dispositif d'ostéosynthèse selon la présente invention utilisable notamment pour les fractures des petits os et destiné au maintien, la distraction ou la compression de fragments d'un os fracturé, comprend une broche filetée comportant à son extrémité distale une tête filetée et une bague adaptée pour coulisser sur la broche filetée. La broche et la bague sont en des matériaux permettant leur sectionnement. La bague comporte à son extrémité distale une tête filetée dont le diamètre est au moins égal au diamètre de la tête filetée de la broche. La broche filetée et la bague filetée sont canulées de façon à permettre leur sertissage par déformation lors du sectionnement.

Préférentiellement, la broche filetée est entièrement canulée.

Selon une variante, la broche filetée est pleine au niveau de la tête filetée.

Avantageusement, la broche et la bague sont réalisées dans des matériaux biocompatibles déformables. Un exemple d'un tel matériau est l'acier inoxydable de type 316LVM, qui peut être écroui pour la broche, de façon à avoir une tension de rupture *Rm* supérieure à 900 MPa, et hypertrempé pour la bague, avec *Rm* ≈ 600 MPa.

Préférentiellement, les filetages des têtes filetées sont identiques.

Selon une variante, les filetages des têtes filetées sont différents.

Avantageusement, lors du sectionnement, les sections de la broche et de la bague sont déformées de façon à ce leurs bords, dans un plan perpendiculaire aux axes de la broche et de la bague, se rapprochent dans une première direction et s'éloignent dans une deuxième direction perpendiculaire à la première direction.

Conformément à l'invention, la broche filetée comporte un corps cylindrique terminé par un filetage distal externe de type à os. La broche filetée peut être entièrement canulée pour être guidée sur broche ou partiellement canulée pour être vissée directement. Le filetage distal externe de la broche comporte des moyens connus favorisant l'auto-taraudage. La bague filetée comporte un corps cylindrique terminé par filetage distal externe de type à os et est entièrement perforée pour coulisser sur le corps cylindrique de la broche filetée. Le filetage distal externe de la bague comporte des moyens connus favorisant l'auto-taraudage

Suivant un mode avantageux de réalisation de l'invention, la longueur des filetages distaux externes est d'environ 5mm.

Les avantages de la présente invention seront plus clairs avec l'explication de pose suivante. Le praticien insère, au moteur ou manuellement à l'aide d'un mandrin « Jacob », la broche filetée partiellement canulée au travers du fragment d'os proximal jusqu'à ce que le filetage distal de la broche filetée s'ancre dans l'os distal. Après avoir désolidarisé le moyen d'entrainement du corps cylindrique de la broche filetée partiellement canulée, il fait coulisser la bague filetée sur le corps cylindrique de la broche filetée partiellement canulée jusqu'au contact osseux puis la visse dans l'os. À ce moment, il peut, en tirant ou en poussant sur le corps de la bague filetée, soit écarter le fragment osseux proximal de l'os distal, soit comprimer le fragment osseux sur l'os. Quand la distraction ou compression souhaitée est effectuée, le praticien, tout en maintenant le montage en place, sectionne l'ensemble au ras de l'os, par exemple au moyen d'une pince coupante. La pince coupante, agissant sur ces deux pièces creuses, déforme les sections des corps de la broche et de la bague avant de les sectionner, réalisant ainsi un sertissage du corps cylindrique canulé de la broche filetée dans le corps cylindrique canulé de la bague filetée. Ce sertissage permet de maintenir le montage dans la position réalisée par le praticien.

Le mode de réalisation selon lequel la broche filetée est entièrement canulée permet au praticien de la mettre en place au moyen d'une broche guide de type « Kirschner » préalablement insérée et sur laquelle la broche filetée peut coulisser. Lorsque la broche filetée entièrement canulée est ancrée dans l'os distal, le praticien retire la broche de « Kirschner » et continue son geste opératoire comme décrit ci-dessus.

On comprendra que le dispositif selon la présente invention fonctionne de la même façon si le fragment osseux est distal par rapport à un os proximal, ou pour solidariser deux ou plusieurs fragments osseux.

L'ablation du dispositif est particulièrement simple. Lors du sertissage, le corps cylindrique canulé de la broche filetée et le corps de la bague filetée se déforment de telle façon qu'il reste une fente permettant l'introduction d'un outil d'entrainement, tel que l'extrémité d'un tournevis, entrainant les deux pièces lors du dévissage.

D'autres caractéristiques et avantages de la présente invention apparaîtront dans la description suivante, relative à un mode préférentiel de réalisation, en référence aux dessins annexés, qui représentent :
Figure 1 : vue en perspective d'une broche filetée partiellement canulée d'un dispositif selon l'invention ;
Figure 2 : vue en perspective de la bague filetée entièrement canulée du dispositif selon l'invention ;
Figure 3 : vue en perspective du dispositif selon l'invention vissé dans un os avant mise en compression ;
Figure 4 : vue en coupe du dispositif selon l'invention vissé dans un os après mise en compression et sectionnement ; et
Figures 5 à 6 : vues illustrant le sertissage du dispositif après sectionnement.

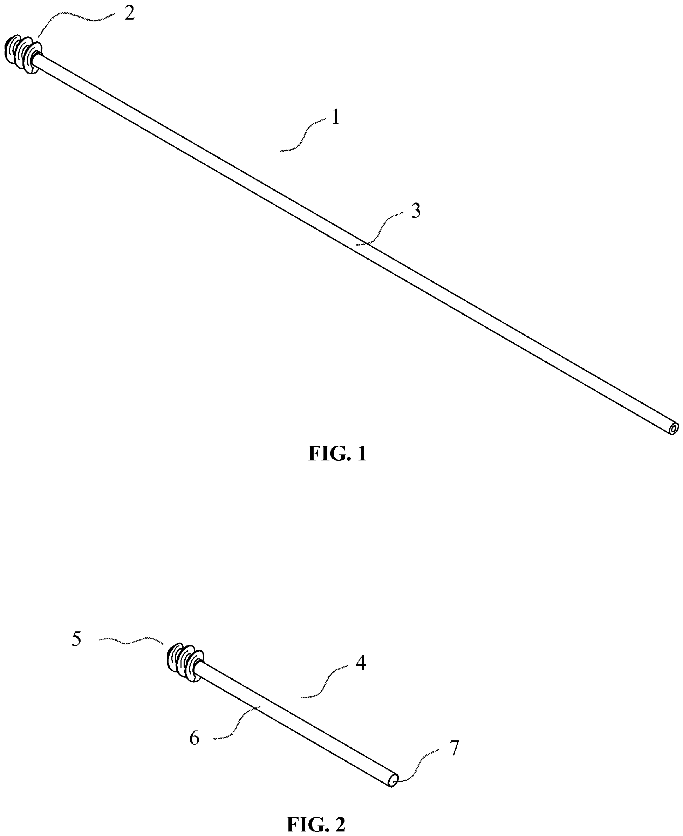

La broche filetée 1, représentée sur la Figure 1, est composée d'une tête 2 prolongée par un corps 3. La tête 2 est filetée suivant un filetage à os connu dont l'extrémité distale peut être de forme pyramidale et/ou comporter des moyens favorisant le taraudage. Le corps 3 est canulé, et la tête 2 est pleine.

Alternativement, la broche filetée 1 peut être entièrement canulée pour être guidée sur broche préalablement positionnée.

La bague filetée 4, représentée sur la Figure 2, comporte une tête 5 prolongée par un corps 6. La tête 5 est filetée suivant un filetage à os connu dont l'extrémité distale peut comporter des moyens favorisant le taraudage. Le filetage de la tête 5 de la bague 4 est préférentiellement identique au filetage de la tête 2 de la broche 1 de façon à se visser facilement dans le taraudage effectué par le passage de la broche filetée 1. La bague filetée 4 est entièrement canulée de façon à pouvoir coulisser sur le corps 3 de la broche 1. Le perçage 7 du corps 6 de la bague 4 est de diamètre légèrement supérieur au diamètre externe du corps 3 de la broche 1.

Selon une variante, le filetage de la tête 2 de la broche 1 est différent de celui de la tête 5 de la bague 4. Ceci est particulièrement avantageux dans le cas où les os ou les fragments d'os dans lesquels les têtes s'ancrent sont de types différents. Par exemple, la broche peut se fixer dans un os cortical et la bague dans un os spongieux, ou vice versa.

Selon des variantes, le diamètre du filetage de la tête 5 de la bague 4 peut être supérieur ou égal au diamètre du filetage de la tête 2 de la broche 1. Ainsi, la tête 5 de la bague 4 n'a pas de jeu dans l'os.

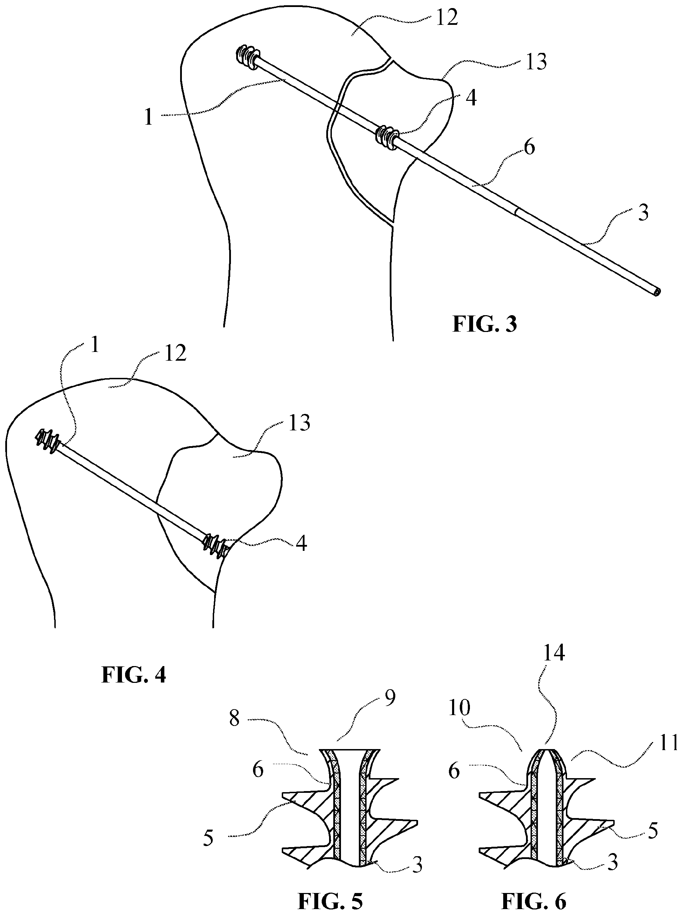

La Figure 3 représente le dispositif, composé de la broche 1 et de la bague 4, vissé dans un os 12 dont la fracture n'est pas réduite. La broche filetée 1 est vissée dans l'os et le corps 3 de la broche 1 dépasse du corps 6 de la bague filetée 4 pour faciliter les manipulations. La bague filetée 4 est vissée dans le fragment osseux 13 et le corps 6 de la bague 4 dépasse du fragment osseux 13 afin de faciliter les manipulations.

La Figure 4 représente le dispositif après réduction de la fracture du fragment osseux 13 et sectionnement du dispositif au ras du fragment osseux 13. Le sectionnement peut être effectué, par exemple, au moyen d'une pince coupante. Sous l'action de la pince coupante au ras, avant le sectionnement, le corps 3 de la broche 1 et le corps 6 de la bague 4 sont déformés générant un sertissage des deux corps 3, 6.

La Figure 5 représente une section, dans un plan donné contenant les axes de la broche 1 et de la bague 4, de la bague filetée 4 et d'une partie du corps 3 de la broche 1 après sectionnement. Le corps 6 de la bague 4 s'est évasé suivant la forme 8 et le corps 3 de la broche 1 s'est évasé suivant la forme 9. La combinaison des deux formes évasées permet de bloquer la translation du corps 3 de la broche 1 vers l'intérieur du corps 6 de la bague 4 et de bloquer en rotation la broche filetée 1 par rapport à la bague filetée 4.

La Figure 6 représente une section, dans un plan perpendiculaire au plan de la Figure 5, de la bague filetée 4 et d'une partie du corps 3 de la broche 1 après sectionnement. Le corps 6 de la bague 4 s'est refermé suivant la forme 10 et le corps 3 de la broche 1 s'est refermé suivant la forme 11. La combinaison des deux formes refermées permet de bloquer la translation du corps 3 de la broche 1 vers l'extérieur du corps 6 de la bague 4 et de bloquer en rotation la broche filetée 1 par rapport à la bague filetée 4.

Ainsi, les déformations des corps 3, 6 de la broche 1 et de la bague 4, décrites en relation aux Figures 5 et 6, bloquent la broche filetée 1 en translation et en rotation par rapport à la bague filetée 4. L'espace 14 créé lors du sectionnement du dispositif par les bords des deux corps 3, 6, visible sur la Figure 6, permet de visser ou de dévisser l'ensemble au moyen d'un tournevis. Par exemple, le praticien peut enfouir l'ensemble dans l'os ou encore procéder à son ablation.

## Revendications

1. Dispositif d'ostéosynthèse destiné au maintien, la distraction ou la compression de fragments d'un os fracturé, comprenant :
- une broche filetée (1) comportant à son extrémité distale une tête filetée (2), et
- une bague (4) adaptée pour coulisser sur la broche filetée (1), dans lequel
- la broche (1) et la bague (4) sont en des matériaux permettant leur sectionnement,
- la bague (4) comporte à son extrémité distale une tête filetée (5) dont le diamètre est au moins égal au diamètre de la tête filetée (2) de la broche (1), et
- la broche filetée (1) et la bague filetée (4) sont canulées,
**caractérisé en ce que**:
- la broche filetée (1) et la bague filetée (4) sont canulées de façon à permettre leur sertissage par déformation lors du sectionnement.

2. Dispositif selon la revendication 1, **caractérisé en ce que** la broche filetée (1) est entièrement canulée.

3. Dispositif selon la revendication 1, **caractérisé en ce que** la broche filetée (1) est pleine au niveau de la tête filetée (2).

4. Dispositif selon l'une des revendications 1 à 3, **caractérisé en ce que** la broche (1) et la bague (4) sont réalisées dans des matériaux biocompatibles déformables.

5. Dispositif selon l'une des revendications précédentes, **caractérisé en ce que** les filetages des têtes filetées (2, 5) sont identiques.

6. Dispositif selon l'une des revendications 1 à 4, **caractérisé en ce que** les filetages des têtes filetées (2, 5) sont différents.

7. Dispositif selon l'une des revendications précédentes, **caractérisé en ce que** lors du sectionnement, les sections de la broche (1) et de la bague (4) sont déformées de façon à ce leurs bords, dans un plan perpendiculaire aux axes de la broche (1) et de la bague (4), se rapprochent dans une première direction et s'éloignent dans une deuxième direction perpendiculaire à la première direction.

8. Dispositif selon l'une des revendications précédentes, **caractérisé en ce que** la longueur de chacun des filetages est de 5 mm.

## Patentansprüche

1. Osteosynthese-Vorrichtung, die zum Halten, zur Distraktion oder zur Kompression von Fragmenten eines gebrochenen Knochens bestimmt ist, umfassend:
- eine Gewindespindel (1), die an ihrem distalen Ende einen Gewindekopf (2) aufweist, und
- einen Ring (4), der ausgebildet ist, um auf der Gewindespindel (1) zu gleiten,
wobei
- die Spindel (1) und der Ring (4) aus Materialien sind, die ihr Schneiden erlauben,
- der Ring (4) an seinem distalen Ende einen Gewindekopf (5) aufweist, dessen Durchmesser mindestens dem Durchmesser des Gewindekopfs (2) der Spindel (1) entspricht, und
- die Gewindespindel (1) und der Gewindering (4) kanüliert sind,
**dadurch gekennzeichnet, dass**
die Gewindespindel (1) und der Gewindering (4) derart kanüliert sind, dass ihr Falzen durch Verformen beim Schneiden möglich ist.

2. Vorrichtung nach Anspruch 1, **dadurch gekennzeichnet, dass** die Gewindespindel (1) vollständig kanüliert ist.

3. Vorrichtung nach Anspruch 1, **dadurch gekennzeichnet, dass** die Gewindespindel (1) im Bereich des Gewindekopfs (2) massiv ist.

4. Vorrichtung nach einem der Ansprüche 1 bis 3, **dadurch gekennzeichnet, dass** die Spindel (1) und der Ring (4) aus verformbaren biologisch verträglichen Materialien hergestellt sind.

5. Vorrichtung nach einem der vorangehenden Ansprüche, **dadurch gekennzeichnet, dass** die Gewinde der Gewindeköpfe (2, 5) identisch sind.

6. Vorrichtung nach einem der Ansprüche 1 bis 4, **dadurch gekennzeichnet, dass** die Gewinde der Gewindeköpfe (2, 5) unterschiedlich sind.

7. Vorrichtung nach einem der vorangehenden Ansprüche, **dadurch gekennzeichnet, dass** beim Schneiden die Schnitte der Spindel (1) und des Rings (4) derart verformt sind, dass sich ihre Ränder in einer zu den Achsen der Spindel (1) und des Ring (4) senkrechten Ebene in einer ersten Richtung annähern und sich in einer zweiten, zur ersten Richtung senkrechten Richtung entfernen.

8. Vorrichtung nach einem der vorangehenden Ansprüche, **dadurch gekennzeichnet, dass** die Länge jedes der Gewinde 5 mm beträgt.

## Claims

1. An osteosynthesis device intended for maintaining, distracting or compressing fragments of a fractured bone, comprising:
- a threaded pin (1) including, at its distal end, a threaded head (2), and
- a ring (4) suitable for sliding on the threaded pin (1),
wherein
- the pin (1) and the ring (4) are made from materials allowing them to be sectioned,
- the ring (4) includes, at its distal end, a threaded head (5) whose diameter is at least equal to the diameter of the threaded head (2) of the pin (1), and
- the threaded pin (1) and the threaded ring (4) are cannulated,
**characterized in that**:
the threaded pin (1) and the threaded ring (4) are cannulated so as to allow them to be crimped by deformation during sectioning.

2. The device according to claim 1, **characterized in that** the threaded pin (1) is completely cannulated.

3. The device according to claim 1, **characterized in that** the threaded pin (1) is solid at the threaded head (2).

4. The device according to one of claims 1 to 3, **characterized in that** the pin (1) and the ring (4) are made from deformable biocompatible materials.

5. The device according to one of the preceding claims, **characterized in that** the threads of the threaded heads (2, 5) are identical.

6. The device according to one of claims 1 to 4, **characterized in that** the threads of the threaded heads (2, 5) are different.

7. The device according to one of the preceding claims, **characterized in that** during the sectioning, the sections of the pin (1) and the ring (4) are deformed such that their edges, in a plane perpendicular to the axis of the pin (1) and the ring (4), come closer together in a first direction and move further apart in a second direction perpendicular to the first direction.

8. The device according to one of the preceding claims, **characterized in that** the length of each of the threads is 5 mm.
